# EUROPÄISCHE PATENTSCHRIFT

(11) **EP 2 420 258 B1**
(45) Veröffentlichungstag und Bekanntmachung des Hinweises auf die Patenterteilung: **12.04.2017**
(21) Anmeldenummer: 11176772.9
(22) Anmeldetag: 08.08.2011
(51) Int. Cl.: A61L 2/232, B65B 55/02, B67C 7/00, A61L 2/238

(54) **Vorrichtung zum Behandeln von Verpackungen**
Device for handling packaging
Dispositif de traitement d'emballages

(30) Priorität: 19.08.2010 DE 102010034895
(43) Veröffentlichungstag der Anmeldung: 22.02.2012
(73) Patentinhaber: Krones AG, 93073 Neutraubling (DE)
(72) Erfinder: Lappe, Ulrich, 93059 Regensburg (DE)
(74) Vertreter: Bittner, Bernhard

(56) Entgegenhaltungen:
- WO-A1-2006/099906
- WO-A2-2009/024218
- WO-A2-2009/115774
- US-A- 6 012 267

## Beschreibung

Die vorliegende Erfindung bezieht sich auf eine Vorrichtung zum Behandeln von Verpackungen, insbesondere von Behältern wie z.B. Vorformlingen oder Behältnissen. Derartige Vorrichtungen sind aus dem Stand der Technik seit langem bekannt. Bei diesen Vorrichtungen kann es sich beispielsweise um Vorrichtungen handeln, welche Kunststoffvorformlinge zu Kunststoffbehältnissen umformen, wie beispielsweise Blasmaschinen. Es kann sich dabei um Sterilisationseinrichtungen handeln, welche Behältnisse oder Kunststoffvorformlinge sterilisieren, um Transporteinrichtungen für Behältnisse bzw. Vorformlinge und dergleichen. Die Erfindung wird insbesondere unter Bezugnahme auf solche Vorrichtungen beschrieben, welche Kunststoffvorformlinge zu Kunststoffbehältnissen umformen. Dabei ist es aus dem Stand der Technik bekannt, dass die Sterilisationseinrichtungen die Kunststoffvorformlinge oder die Behältnisse sterilisieren. Der Kunststoffvorformling ist nach diesem Prozess keimreduziert bzw. frei von Produktschädlingen. Der darauffolgende Blasprozess und die Kontakte der Maschinenteile, wie beispielsweise bei Übergaben, aber auch die Reckstange, welche die Kunststoffvorformlinge dehnt oder die Blasdüse bergen jedoch die Gefahr, die Kunststoffvorformlinge oder auch die Behältnisse wieder mit Produktschädlingen zu kontaminieren.

WO2009024218 offenbart eine Vorrichtung zur H2O2-Sterilisation von Packmitteln, mit wenigstens einem das Wasserstoffperoxid bereitstellenden Vorratsbehälter und mit einer steuerbaren Verbindung zwischen dem wenigstens einem Vorratsbehälter und dem jeweiligen Behandlungskopf, dabei ist vorgesehen, dass die steuerbare Verbindung von wenigstens einer Ringleitung gebildet ist, die zusammen mit dem wenigstens einen Vorratsbehälter einen Kreis- oder Umlauf für das Wasserstoffperoxid bildet, und dass der jeweilige Behandlungskopf über wenigstens ein Dosierventil an die wenigstens eine Ringleitung angeschlossen ist.

WO2009115774 offenbart einen Flaschenfüller mit partieller antimikrobieller Beschichtung aus Silber oder Titandioxid.

US6012267 offenbart eine Verpackungsmaschine, wobei wasserkontaktierte Flächen aus einer Kupfer-Edelstahllegierung bestehen, oder mit einem Fotokatalysator beschichtet sind.

Der vorliegenden Erfindung liegt daher die Aufgabe zugrunde, die Gefahr derartiger Rekontaminationen zu verringern. Diese Aufgabe wird durch die Gegenstände der unabhängigen Ansprüche erreicht. Vorteilhafte Ausführungsformen und Weiterbildungen sind Gegenstand der Unteransprüche.

Eine erfindungsgemäße Vorrichtung zum Behandeln von Behältern wird in Anspruch 1 beschrieben. Unter einer keimabtötenden Ausgestaltung bzw. Modifikation wird verstanden, dass die besagte Oberfläche insbesondere einer Behandlung unterzogen wurde, welche sie derart modifiziert, dass auf diese Oberfläche gelangende Keime abgetötet oder allgemein unschädlich gemacht werden bzw. ihre Vermehrung unterbunden wird.

Allgemein sollten sämtliche Maschinenteile, die in Kontakt mit den Kunststoffbehältnissen oder den Kunststoffvorformlingen kommen an der Oberfläche derart modifiziert werden, dass diese eine keimtötende Wirkung haben.

Unter den Kunststoffbehältern werden dabei nicht nur die eigentlichen Behälter verstanden sondern auch deren Bestandteile wie insbesondere deren Verschlüsse. Damit kann die Erfindung auch auf solche Kontaktflächen Anwendung finden, welche mit einer Oberfläche der Behälterverschlüsse und insbesondere der Innenoberfläche der Verschlüsse in Kontakt stehen. Bei entsprechenden Vorrichtungen kann es sich beispielsweise um Verschliesser handeln, welche die Behälter mit Verschlüssen verschließen. Bei dem Behandlungselement kann es sich dabei um einen den Behälterverschluss aufnehmenden Verschliesserkopf handeln, der den Verschluss beispielsweise auf den Behälter aufschraubt. Bei einer Transporteinrichtung kann es sich um eine Transporteinrichtung zum Transport der Verschlüsse handeln.

Bei einer bevorzugten Ausführungsform weist wenigstens eine Kontaktfläche eine keimabtötende Beschichtung auf. Dabei kann beispielsweise eine Oberfläche eine Silberionenbeschichtung aufweisen. Die Verwendung von Silberionenbeschichtungen wurde im Stand der Technik bereits beschrieben. So wurde beispielsweise in der DE 10 2007 025 452 A1 ein Verfahren zur Beschichtung von Oberflächen mit Mikro- und Nanopartikeln mit Hilfe von Plasmaverfahren beschrieben.

Bei einer vorteilhaften Ausführungsform weist die Vorrichtung einen Reinraum auf, innerhalb dessen die Behälter transportiert werden. So ist es möglich, dass die Behälter innerhalb dieses Reinraums mit einem Getränk befüllt werden, innerhalb dieses Reinraums Kunststoffvorformlinge zu den Behältern umgeformt werden, ggfs. sogar innerhalb des besagten Reinraums die Kunststoffvorformlinge erwärmt werden, bevor sie in einer nachfolgenden Blasmaschine umgeformt werden. Dieser Reinraum oder Sterilraum dient dabei dazu, um die entsprechende Behälterbehandlung unter sterilen Bedingungen durchzuführen.

Bei einer weiteren vorteilhaften Ausführungsform ist das Transportelement aus einer Gruppe von Transportelementen ausgewählt, wie Greifklammern, Heizdorne, Sortierrollen, Scheiben eines Scheibensortierers und dergleichen.

Vorteilhaft weist die Transporteinrichtung eine Vielzahl von Transportelementen auf, welche gleichzeitig eine Vielzahl von Behältern transportieren, wobei hierbei die Behälter bevorzugt vereinzelt transportiert werden.

Weiterhin kann es sich auch um Transportelemente handeln, welche den Behälter zu dessen Transport an dessen Mündungsbereich kontaktieren, wie beispielsweise Greifklammern, welche unterhalb eines Tragrings des Kunststoffvorformlings oder des Kunststoffbehälters eingreifen.

Bei einer weiteren vorteilhaften Ausführungsform ist das Behandlungselement aus einer Gruppe von Behandlungselementen ausgewählt, welche Blasdüsen, Reckstangen, Blasformen, Heizdorne, Stützringe und dergleichen enthält. So kontaktiert beispielsweise eine Blasdüse bei vielen Vorrichtungen einen Mündungsbereich der Behältnisse und ist aus diesem Grund zumindest in dieser Kontaktfläche mit der Mündung der Behältnisse in keimabtötender Weise behandelt. Im Falle der Reckstangen ist es insbesondere möglich, dass deren Endabschnitte bzw. deren Spitzen die Innenwandung der Kunststoffvorformlinge kontaktieren, sodass vorteilhaft wenigstens die Spitzen in keimabtötender Weise modifiziert sind. Die Stützringe können beispielsweise während der Expansion der Kunststoffvorformlinge diese Kunststoffvorformlinge gegenüber einer Blasform abstützen. Die Blasformen kontaktieren insbesondere eine Außenwand des Kunststoffvorformlings während dessen Expansion.

Bei einer weiteren vorteilhaften Ausführungsform weist die Vorrichtung eine Sterilisationseinrichtung auf, welche die Behälter wenigstens abschnittsweise sterilisiert. Dabei ist es möglich, dass die Behälter mit einem Sterilisationsmittel beaufschlagt werden, es wäre jedoch auch eine Sterilisation der Behälter beispielsweise durch Elektronenstrahlen (E-beam) oder durch UV-Bestrahlung und dergleichen denkbar. Auch Kombinationen aus mehreren Sterilisationsverfahren wären denkbar. Als Sterilisationsmittel kommen dabei beispielsweise Wasserstoffperoxid oder auch Peressigsäure in Frage.

Bei einer vorteilhaften Ausführungsform ist wenigstens eine Kontaktfläche in der Transportrichtung der Behälter stromabwärts bezüglich der besagten Sterilisationseinrichtung angeordnet. Dies bedeutet, dass nach der Sterilisation der Behälter oder auch der Kunststoffvorformlinge eine erneute Rekontamination der Behälter vermieden wird. So ist es möglich, dass nach der besagten Sterilisationseinrichtung die Behälter nur noch über Kontaktflächen kontaktiert werden, die besagte keimabtötende Wirkung aufweisen. Dies kann beispielsweise so lange erfolgen, bis der Behälter befüllt und mit einem Verschluss verschlossen ist.

Bei einer weiteren vorteilhaften Ausführungsform weist die Vorrichtung eine Umformungseinrichtung zum Umformen von Kunststoffvorformlingen zu Kunststoffbehältern auf.

Bei einer weiteren vorteilhaften Ausführungsform kann die Vorrichtung auch eine Anlage zum Füllen von Behältern mit einer Flüssigkeit und insbesondere einem Getränk aufweisen.

Bei einer weiteren vorteilhaften Ausführungsform sind in einem vorgegebenen Transportbereich der Behälter sämtliche Kontaktflächen, welche mit den Behältern in Kontakt treten, keimabtötend modifiziert.

Bei einer weiteren vorteilhaften Ausführungsform handelt es sich bei wenigstens einer Kontaktfläche um eine solche Kontaktfläche, welche eine Innenwandung der Behälter oder einen Gewindebereich des Kunststoffvorformlings kontaktiert. Dies gilt insbesondere für bestimmte Greifklammern oder etwa die oben erwähnte Reckstange oder die Blasdüsen.

Die vorliegende Erfindung ist weiterhin auf ein Verfahren zum Behandeln von Behältern nach Anspruch 8 gerichtet.

Vorzugsweise werden die Kunststoffbehältnisse bzw. Kunststoffvorformlinge sterilisiert und bevorzugt sind zumindest diejenigen Kontaktflächen keimabtötend ausgestaltet, welche in der Transportrichtung der Behältnisse diese Behältnisse nach deren Sterilisation kontaktieren
Weitere Vorteile und Ausführungsformen ergeben sich aus den beigefügten Figuren: Dabei zeigt:
- Fig. 1: eine grob schematische Darstellung einer erfindungsgemäßen Anlage; und
- Fig. 2: eine Darstellung zur Veranschaulichung der Kontaktflächen

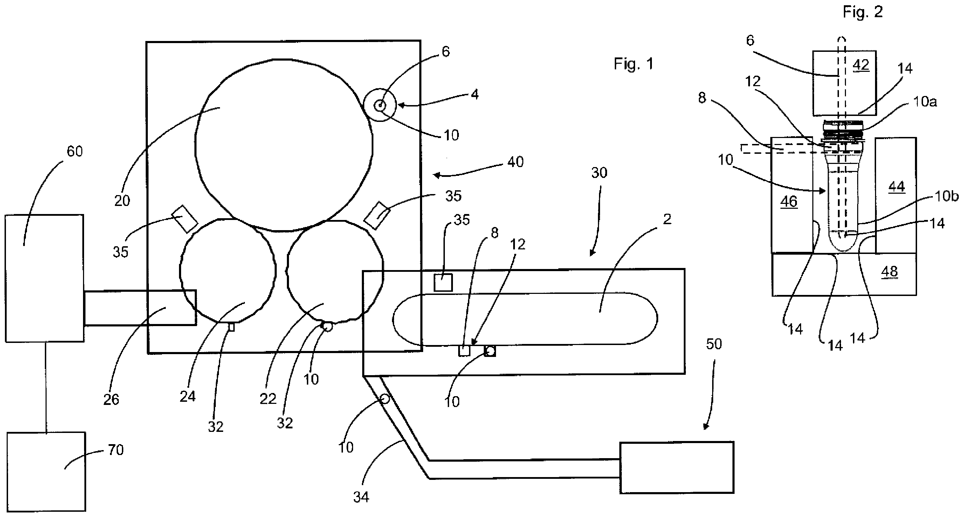

Fig. 1 zeigt eine grob schematische Darstellung einer erfindungsgemäßen Vorrichtung 1. Diese Vorrichtung 1 weist eine Heizeinrichtung 30 auf, welche von Kunststoffvorformlingen 10 durchlaufen wird wobei diese erwärmt werden. Dabei weist die Heizeinrichtung 30 eine Transporteinrichtung 2 und eine Vielzahl von Transportelementen 8 auf, welche die Kunststoffvorformlinge 10 vereinzelt transportieren. Bei diesen Transportelementen kann es sich beispielsweise um Dorne handeln, welche in eine Mündung der Kunststoffbehältnisse 10 eingreifen. Die Außenwandung dieser Dorne stellt in diesem Falle die Kontaktfläche 12 mit den Kunststoffvorformlingen dar. Daneben kann es sich bei den Transportelementen auch um Klammern handeln, welche die Kunststoffvorformlinge unterhalb deren Tragring greifen. In diesem Fall könnten die Innenoberflächen dieser Greifklammern, welche die Kunststoffvorformlinge kontaktieren, in keimabtötender Weise modifiziert sein. Als Transporteinrichtung kann beispielsweise eine Transportkette vorgesehen sein, an der eine Vielzahl von Transportelementen angeordnet ist.

Nach Durchlaufen der Heizeinrichtung 30 werden die Kunststoffvorformlinge über eine weitere Transporteinrichtung 22 an eine Umformungseinheit 40, genauer gesagt, ein Blasrad 20 dieser Umformungseinheit 40 übergeben. Dieses (um eine vorgegebene Drehachse drehbare) Blasrad 20 weist dabei eine Vielzahl von Behandlungseinrichtungen 4 (nur eine gezeigt), wie beispielweise Blasstationen auf, innerhalb denen die Kunststoffvorformlinge 10 durch Beaufschlagung mit Druckluft zu Kunststoffbehältnissen expandiert werden.

Das Bezugszeichen 6 bezieht sich auf ein Behandlungselement, wie beispielsweise eine Reckstange, welche die Kunststoffvorformlinge in deren Längsrichtung dehnt. Nach dem Umformungsprozess werden die Kunststoffvorformlinge mit einer weiteren Transporteinrichtung 24, welche eine Vielzahl von Transportelementen 32 aufweist, transportiert.

Das Bezugszeichen 26 bezieht sich auf eine weitere Transporteinrichtung, welche die umgeformten Behälter an eine Befüllungseinrichtung 60 (nur schematisch dargestellt) übergibt. Ein Element dieser Befüllungseinrichtung 60, wie beispielsweise ein Befüllungselement, kann dabei ebenfalls keimabtötend gestaltete Kontaktflächen aufweisen. An die Befüllungseinrichtung schließt sich eine (ebenfalls nur schematisch dargestellte) Verschließereinrichung 70 an, welche die Kunststoffvorformlinge mit Verschlüssen schließt. Dabei können auch Transporteinrichtungen, welche diese Verschlüsse transportieren, an Oberflächen keimabtötend modifiziert sein. Auch können Behandlungselemente, wie etwa Verschließköpfe in entsprechender Weise, wie oben erwähnt, keimabtötend modifiziert sein.

Das Bezugszeichen 50 bezieht sich auf ein Reservoir, welches die Kunststoffvorformlinge 10 zur Verfügung stellt. Dabei können die Kunststoffvorformlinge beispielsweise über eine Transporteinrichtung 34 wie einen Rollensortierer gefördert werden. Dabei wäre es möglich, dass auch die Rollen dieses Rollensortierers 34 keimabtötend an ihren Oberflächen ausgestaltet sind.

Neben oder anstelle des Reservoirs könnte auch eine Formmaschine zum Herstellen der Kunststoffvorformlinge vorhanden sein. Weiterhin weist die dargestellte Anlage zusätzlich einen Reinraum auf, durch welchen hindurch die Kunststoffvorformlinge transportiert werden. Dabei wäre es möglich, dass nur einzelne Bereiche der Anlage einen entsprechenden Reinraum aufweisen, etwa alle Anlagenteile ab der Umformungseinheit. Es wäre jedoch auch möglich, dass ein derartiger Reinraum bereits vor der Heizeinrichtung 30 beginnt und sich durchgehend bis zum Ende der Befüllungseinrichtung erstreckt, so dass sichergestellt wird, dass die Behälter innerhalb des Reinraums erwärmt und umgeformt und auch befüllt werden.

Das Bezugszeichen 35 kennzeichnet Sterilisationseinrichtungen zum Sterilisieren der Kunststoffvorformlinge. Diese Sterilisationseinrichtungen können beispielsweise an der Transporteinrichtung 22 und/oder der Heizeinrichtung 30 angeordnet sein. Auch wäre es möglich, dass mehrere derartige Sterilisationseinrichtungen 35 vorgesehen sind. Daneben können auch Sterilisationseinrichtungen zum Sterilisieren der Behälterverschlüsse vorgesehen sein.

Fig. 2 zeigt eine schematische Darstellung zur Veranschaulichung möglicher Kontaktflächen. Dabei ist wiederum ein Kunststoffvorformling 10 darstellt, und eine Blasform, die zwei Seitenteile 44 und 46, sowie ein Bodenteil 48 aufweist. Daneben ist auch ein Transportelement 8 dargestellt, sowie eine Reckstange 6, wobei einzelne Bestandteile den Kunststoffvorformling in der Regel nicht zeitgleich, sondern zeitlich versetzt, kontaktieren. Die jeweiligen Bestandteile 44, 46 und 48 der Blasform weisen dabei Kontaktflächen 14 auf, welche einen Grundkörper bzw. eine Außenwandung 10b des Kunststoffvorformlings 10 kontaktieren.

Das Bezugszeichen 12 kennzeichnet eine Kontaktfläche des Transportelements, welche beispielsweise unterhalb eines Gewindes 10a des Kunststoffvorformlings anliegen kann, um diesen zu halten. Die Kontaktflächen können jeweils (nicht genauer dargestellte) keimabtötende Beschichtungen aufweisen.

Das Bezugszeichen 42 kennzeichnet ebenfalls grob schematisch eine Blasdüse, die entsprechend noch eine Kontaktfläche 14 aufweist, mit der sie einen oberen Rand des Kunststoffvorformlings 10 kontaktiert. Die Reckstange 6 weist ebenfalls eine Kontaktfläche 14, insbesondere an ihrem unteren Rand auf, welche die Innenwandung des Kunststoffvorformlings 10 kontaktiert.

Wie ausgeführt handelt es sich bei den Verpackungen insbesondere um Kunststoffvorformlinge. Die Erfindung wäre jedoch auch auf andere Verpackungsmittel anwendbar wie beispielsweise auf Kunststoffflaschen, Glasflaschen, Kartonagen, Verschlüsse und dergleichen.

### Bezugszeichenliste

- 1: Vorrichtung
- 2: Transporteinrichtung
- 4: Behandlungseinrichtung
- 6: Behandlungselement, Reckstange
- 8: Transportelement
- 10: Kunststoffvorformlinge
- 10a: Gewinde des Kunststoffvorformlings
- 10b: Außenwandung des Kunststoffvorformlings
- 12: Kontaktfläche
- 14: Kontaktfläche
- 20: Blasrad
- 22, 24, 26: Transporteinrichtung
- 30: Heizeinrichtung
- 32: Transportelement
- 34: Transporteinrichtung, Rollensortierer
- 35: Sterilisationseinrichtung
- 40: Umformungseinheit
- 42: Blasdüse
- 44, 46: Seitenteil
- 48: Bodenteil
- 50: Reservoir
- 60: Befüllungseinrichtung
- 70: Verschließereinrichtung

## Patentansprüche

1. Vorrichtung (1) zum Behandeln von Behältern (10) mit wenigstens einer Transporteinrichtung (2), welche die Behälter (10) entlang eines vorgegebenen Transportpfades transportiert, wobei die Transporteinrichtung (2) ein Transportelement (8) aufweist, welches wenigstens einen Bereich der Behälter (10) während des Transports über eine erste Kontaktfläche (12) kontaktiert, und mit wenigstens einer Behandlungseinrichtung (4), welche die Behälter (10) in einer vorgegebenen Weise behandelt, wobei die Behandlungseinrichtung (4) ein Behandlungselement (6) aufweist, welches wenigstens zeitweise über eine zweite Kontaktfläche (14) mit einer Oberfläche der Behälter (10) in Kontakt steht,
**dadurch gekennzeichnet, dass**
wenigstens eine Kontaktfläche (12, 14) eine keimabtötende Beschichtung aufweist, wobei die Vorrichtung (1) einen Reinraum aufweist, innerhalb dessen die Behälter (10) transportiert werden und das Transportelement (8) aus einer Gruppe von Transportelementen ausgewählt ist, welche Greifklammern, Heizdorne und Sortierrollen enthält.

2. Vorrichtung (1) nach Anspruch 1,
**dadurch gekennzeichnet, dass**
wenigstens eine Kontaktfläche (12, 14) eine Silberionenbeschichtung aufweist.

3. Vorrichtung (1) nach wenigstens einem der vorangegangenen Ansprüche,
**dadurch gekennzeichnet, dass**
das Behandlungselement (6) aus einer Gruppe von Behandlungselementen ausgewählt ist, welche Blasdüsen, Reckstangen, Blasformen, Heizdorne und Stützringe enthält.

4. Vorrichtung (1) nach wenigstens einem der vorangegangenen Ansprüche,
**dadurch gekennzeichnet, dass**
die Vorrichtung (1) eine Sterilisationseinrichtung (35) aufweist, welche die Behälter wenigstens abschnittsweise sterilisiert.

5. Vorrichtung (1) nach Anspruch 4,
**dadurch gekennzeichnet, dass**
wenigstens eine Kontaktfläche (12, 14) in der Transportrichtung der Behälter (10) stromabwärts bezüglich der Sterilisationseinrichtung (35) angeordnet ist.

6. Vorrichtung (1) nach wenigstens einem der vorangegangenen Ansprüche,
**dadurch gekennzeichnet, dass**
die Vorrichtung (1) eine Umformungseinrichtung (40) zum Umformen von Kunststoffvorformlingen (10) zu Kunststoffbehältnissen (10) aufweist.

7. Vorrichtung (1) nach wenigstens einem der vorangegangenen Ansprüche,
**dadurch gekennzeichnet, dass**
in einem vorgegebenen Transportbereich der Behälter (10) sämtliche Kontaktflächen (12, 14), welche mit den Behältern (10) in Kontakt treten, keimabtötend modifiziert sind.

8. Verfahren zum Behandeln von Behältnissen (10) wobei die Behälter (10) mittels Transportelementen (8) einer ersten Transporteinrichtung (2) transportiert werden und mittels Behandlungselementen (6) einer Behandlungseinrichtung (4) behandelt werden und wobei die Transportelemente (8) mittels einer ersten Kontaktfläche (12) zeitweise mit dem Behälter (10) in Kontakt stehen und die Behandlungselemente (6) der Behandlungseinrichtung (4) zeitweise mittels einer zweiten Kontaktfläche (14) mit dem Behälter (10) in Kontakt stehen,
**dadurch gekennzeichnet, dass**
wenigstens eine Kontaktfläche (12, 14) eine keimabtötende Beschichtung aufweist und die Behälter (10) innerhalb eines Reinraums transportiert werden und das Transportelement (8) aus einer Gruppe von Transportelementen ausgewählt ist, welche Greifklammern, Heizdorne und Sortierrollen enthält.

## Claims

1. An apparatus (1) for treating containers (10), comprising at least one transport unit (2) which transports the containers (10) along a specified transport path, said transport unit (2) including a transport element (8) which contacts at least an area of the containers (10) during transport via a first contact surface (12), and comprising at least one treatment unit (4) which treats the containers (10) in a specified manner, said treatment unit (4) having a treatment element (6) which is in contact, at least temporarily, with a surface of the container (10) via a second contact surface (14),
**characterised in that**
at least one contact surface (12, 14) has a germicidal coating, wherein the apparatus (1) comprises a clean room inside which the containers (10) are transported and the transport element (8) is selected from a group of transport elements which includes grippers, heating mandrels and sorting rollers.

2. The apparatus (1) as claimed in claim 1,
**characterised in that**
at least one contact surface (12, 14) has a silver ion coating.

3. The apparatus (1) as claimed in at least one of the preceding claims,
**characterised in that**
the treatment element (6) is selected from a group of treatment elements including blow nozzles, stretching rods, blow moulds, heating mandrels and support rings.

4. The apparatus (1) as claimed in at least one of the preceding claims,
**characterised in that**
the apparatus (1) includes a sterilisation unit (35) that sterilises the containers at least in sections.

5. The apparatus (1) as claimed in claim 4,
**characterised in that**
at least one contact surface (12, 14) is arranged downstream in relation to the sterilisation unit (35) in the transport direction of the container (10).

6. The apparatus (1) as claimed in at least one of the preceding claims,
**characterised in that**
the apparatus (1) includes a moulding unit (40) for moulding plastic preforms (10) into plastic containers (10).

7. The apparatus (1) as claimed in at least one of the preceding claims,
**characterised in that**
in a specified transport area of the containers (10), all the contact surfaces (12, 14) that come into contact with the containers (10) have been modified so as to be germicidal.

8. A method for treating containers (10), said containers (10) being transported by transport elements (8) of a first transport unit (2) and being treated by treatment elements (6) of a treatment unit (4), and wherein the transport elements (8) are temporarily in contact with the container (10) via a first contact surface (12) and the treatment elements (6) of the treatment unit (4) are temporarily in contact with the container (10) via a second contact surface (14),
**characterised in that**
at least one contact surface (12, 14) has a germicidal coating and the containers (10) are transported inside a clean room and the transport elements (8) are selected from a group of transport elements which includes grippers, heating mandrels and sorting rollers.

## Revendications

1. Dispositif (1) servant à traiter des contenants (10) comprenant au moins un système de transport (2), qui transporte les contenants (10) le long d'un chemin de transport prédéfini, dans lequel le système de transport (2) présente un élément de transport (8), qui établit un contact avec au moins une zone des contenants (10) au cours du transport par l'intermédiaire d'une première surface de contact (12), et comprenant au moins un système de traitement (4), qui traite d'une manière prédéfinie les contenants (10), dans lequel le système de traitement (4) présente un élément de traitement (6), qui se trouve en contact au moins par intermittence avec une surface des contenants (10) par l'intermédiaire d'une deuxième surface de contact (14),
**caractérisé en ce**
**qu'**au moins une surface de contact (12, 14) présente un revêtement germicide, dans lequel le dispositif (1) présente une salle blanche, à l'intérieur de laquelle les contenants (10) sont transportés, et que l'élément de transport (8) est choisi parmi un groupe d'éléments de transport, qui contient des pinces de préhension, des mandrins de chauffage et des galets de tri.

2. Dispositif (1) selon la revendication 1,
**caractérisé en ce**
**qu'**au moins une surface de contact (12, 14) présente un revêtement d'ions d'argent.

3. Dispositif (1) selon au moins l'une quelconque des revendications précédentes,
**caractérisé en ce**
**que** l'élément de traitement (6) est choisi parmi un groupe d'éléments de traitement, qui contient des buses de soufflage, des tiges d'étirage, des moules de soufflage, des mandrins de chauffage et des bagues de soutien.

4. Dispositif (1) selon au moins l'une quelconque des revendications précédentes,
**caractérisé en ce**
**que** le dispositif (1) présente un système de stérilisation (35), qui stérilise au moins par endroits les contenants.

5. Dispositif (1) selon la revendication 4,
**caractérisé en ce**
**qu'**au moins une surface de contact (12, 14) est disposée dans la direction de transport des contenants (10) en aval par rapport au système de stérilisation (35).

6. Dispositif (1) selon au moins l'une quelconque des revendications précédentes,
**caractérisé en ce**
**que** le dispositif (1) présente un système de mise en forme (40) servant à mettre en forme des ébauches de plastique (10) en des récipients en plastique (10).

7. Dispositif (1) selon au moins l'une quelconque des revendications précédentes,
**caractérisé en ce**
**que** toutes les surfaces de contact (12, 14) qui entrent en contact avec les contenants (10) sont modifiées de manière à exercer une action germicide dans une zone de transport prédéfinie des contenants (10).

8. Procédé servant à traiter des récipients (10), dans lequel les contenants (10) sont transportés au moyen d'éléments de transport (8) d'un premier système de transport (2) et sont traités au moyen d'éléments de traitement (6) d'un système de traitement (4) et dans lequel les éléments de transport (8) sont en contact au moyen d'une première surface de contact (12) par intermittence avec le contenant (10) et que les éléments de traitement (6) du système de traitement (4) sont en contact par intermittence au moyen d'une deuxième surface de contact (14) avec le contenant (10),
**caractérisé en ce**
**qu'**au moins une surface de contact (12, 14) présente un revêtement germicide, et en ce que les contenants (10) sont transportés à l'intérieur d'une salle blanche, et en ce que l'élément de transport (8) est choisi parmi un groupe d'éléments de transport, qui contient des pinces de préhension, des mandrins de chauffage et des galets de tri.
